# EUROPÄISCHE PATENTSCHRIFT

(11) **EP 3 145 437 B1**
(45) Veröffentlichungstag und Bekanntmachung des Hinweises auf die Patenterteilung: **10.03.2021**
(21) Anmeldenummer: 15723249.7
(22) Anmeldetag: 20.05.2015
(51) Int. Cl.: A61C 7/00, A61C 7/10

(54) **KIEFERORTHOPÄDISCHE APPARATUR UND VERFAHREN ZUR HERSTELLUNG EINER KIEFERORTHOPÄDISCHEN APPARATUR**
ORTHODONTIC APPARATUS AND METHOD FOR PRODUCING AN ORTHODONTIC APPARATUS
APPAREIL ORTHODONTIQUE ET PROCÉDÉ DE FABRICATION D'UN APPAREIL ORTHODONTIQUE

(30) Priorität: 23.05.2014 CH 787142014
(43) Veröffentlichungstag der Anmeldung: 29.03.2017
(73) Patentinhaber: Digital Smile GmbH, 9413 Oberegg (CH)
(72) Erfinder: GRAF, Simon, CH-3012 Bern (CH); KABASHI, Mark, CH-9434 Au (CH); KISS, Roland, CH-9434 Au (CH)
(74) Vertreter: BOVARD AG
(86) Internationale Anmeldenummer: PCT/EP2015/061156
(87) Internationale Veröffentlichungsnummer: WO 2015/177235

(56) Entgegenhaltungen:
- WO-A1-2014/008583
- WO-A2-2006/125072
- US-A- 5 242 304
- US-A1- 2011 247 214

## Beschreibung

Die vorliegende Erfindung betrifft eine kieferorthopädische Apparatur und ein Verfahren zur Herstellung einer kieferorthopädischen Apparatur, wie etwa eine Zahnspange.

In herkömmlicher Weise werden Zahnspangen hergestellt, indem ein physikalisches Modell eines Kiefers inklusive der Zähne erstellt wird, kieferorthopädisch wirksame Elemente, wie etwa Drähte, auf dem Modell platziert werden und anschliessend ein aushärtender Kunststoff am Modell und den Elementen angeformt wird, der die Elemente fixiert und verbindet. Anschliessend kann ein Nacharbeiten oder Polieren erforderlich sein. Ein solches Verfahren ist z. B. in der DE 3831292 A1 beschrieben.

Für ein solches Verfahren muss zunächst am Patienten eine Abformung des Kiefers mit Zähnen erstellt werden, der als Negativvorlage für das physikalische Kiefermodel dient. Hierfür muss eine Abformungshilfe, z. B. ein Abformlöffel mit Formmasse, wie etwa Gips, Alginat oder Silikon, in den Mund eines Patienten eingeführt, am Kiefer angedrückt und nach einer Wartezeit wieder entnommen werden. Dieses Prozedere ist für den Patienten unangenehm und zeitaufwendig. Ferner müssen die einzelnen Schritte bei der Herstellung der Zahnspange sukzessive in mehreren Schritten unabhängig voneinander durchgeführt werden, wodurch jeweils Wartezeiten entstehen. Die Herstellung der Zahnspange ist daher zeit- und kostenaufwendig. Zudem können bei der Herstellung des Kiefermodells, beim Platzieren der kieferorthopädisch wirksamen Elemente und/oder beim Aushärten des Kunststoffes Toleranzen relativ zum Kiefer des Patienten entstehen, die einen optimalen Sitz der Zahnspange und eine optimale Wirkung verunmöglichen.

Für die Herstellung von Zahnersatzteilen, wurden bildgebende Verfahren entwickelt, aus welchen ein drei-dimensionales Modell des Kiefers mit Zähnen erstellt werden kann. Hierfür wird z. B. eine Kamera oder ein Scanner in den Mund eingeführt, die den Kiefer aus unterschiedlichen Bildwinkeln ablichten. Oder mittels einer anderen drei-dimensionalen bildgebenden Technologie, wie z. B. digitale Volumen-Tomographie, Computer-Tomographie oder Magnet-Resonanz-Tomographie. Eine Computer-Software kann aus diesen Aufnahmen einen Datensatz für ein virtuelles Kiefermodell errechnen, aus welcher ein weiterer Datensatz für eine Formgebung des Zahnersatzteils ermittelt wird. Auf der Grundlage dieses Datensatzes kann z. B. mittels Rapidprototyping-Verfahren das Zahnersatzteil hergestellt werden. Dabei wird das Zahnersatzteil durch schrittweises Aushärten von aufeinander aufgebrachten Materialschichten z. B. mittels Laserstrahlung aufgebaut. Solche Verfahren sind z. B. in der DE 102007014985 A1 und der DE 102005049886 A1 beschrieben. Zahnersatzteile, wie etwa Zahnkronen, Zahnbrücken oder Implantate, sind raumausfüllende Einzelteile mit einem Volumen von mehreren Quadratmillimetern. Für derart voluminöse Teile sind additive Verfahren, wie das Rapidprototyping, aus der mechanischen Produktionsindustrie bekannt.

Aus WO 2014/008583 ist ein Retainer bekannt, welcher derart gestaltet und gefertigt ist, dass er direkt an dem Hinterschnitt der Zähne anliegt und mittels Halteelementen lösbar an den jeweiligen Backenzähnen verankert ist.

Es ist eine Aufgabe der Erfindung eine kieferorthopädische Apparatur zu schaffen, die für einen Patienten angenehm zu tragen ist, die eine kieferorthopädische Behandlung einwandfrei umsetzt und in einfacher Weise anzupassen ist. Weiter ist es eine Aufgabe der Erfindung ein Verfahren zur Herstellung einer kieferorthopädischen Apparatur bereitzustellen, das den Patienten nur wenig belastet, mit wenigen Arbeitsschritten auskommt, schnell und präzise ist, sowie Anpassungen in einfacher Weise ermöglicht.

Diese Aufgabe wird von der Erfindung durch eine kieferorthopädische Apparatur nach Anspruch 1 und ein Verfahren zur Herstellung einer kieferorthopädischen Apparatur nach Anspruch 6 gelöst. Vorteilhafte Ausgestaltungen und weitere Ausführungsbeispiele sind in den abhängigen Ansprüchen beschrieben.

Eine kieferorthopädische Apparatur nach der vorliegenden Erfindung weist wenigstens ein kieferorthopädisch wirksames Elemente, ein als vorgefertigtes Bauelement ausgebildetes Verstellelement, z.B. zum Einstellen eines Drahtbügels und wenigstens ein Halteelement zum Halten der Apparatur am Kiefer auf. Ein kieferorthopädisch wirksames Element ist z. B. ein Drahtbügel, der z. B. an einem oder mehreren Zähnen anliegt, eine Kieferplatte, die am Kiefer anliegt, etc. und andere Elemente, wie sie bei kieferorthopädischen Apparaturen üblich sind. Ein Halteelement kann z. B. ein Zahnaufsatz sein, der auf oder um einen Zahn gelegt wird, um die Apparatur am Kiefer zu fixieren. Grundsätzlich kann ein Halteelement auch durch ein kieferorthopädisch wirksames Elemente gegeben sein. In der Regel weist die Apparatur mehrere kieferorthopädisch wirksame Elemente und mehrere Haltelemente auf, um individuell auf unterschiedliche Bereiche im Kiefer einwirken zu können und die Apparatur sicher am Kiefer halten zu können.

Nach der Erfindung ist die kieferorthopädische Apparatur einstückig ausgebildet. Unter dem Begriff "einstückig" soll dabei verstanden werden, dass die gesamte Apparatur mit all ihren Elementen in einem Stück geformt ist. Erfindungsgemäss sind das wenigstens eine kieferorthopädisch wirksame Element und das wenigstens eine Halteelement aus einem Fertigungsmaterial gefertigt, welches schichtweise um das Verstellelement verteilt und gehärtet ist, welches als vorgefertigtes Bauelement aus einem anderen Fertigungsmaterial gefertigt ist. Die einstückige Apparatur weist somit verschiedene Bereiche auf, welche den kieferorthopädisch wirksamen Elementen, dem Verstellelement und den Halteelementen entsprechen und deren Funktion erfüllen. Die verschiedenen Bereiche gehen unmittelbar in einander über. Dabei ist es durchaus gemäss einer Ausführungsform möglich, dass verschiedene Bereiche der Apparatur aus unterschiedlichen Materialien bestehen, beispielsweise aus Metall oder Kunststoff, die ohne weitere Verbindungmittel aneinander geformt werden. Aber verschiedene Bereiche können auch aus gleichen Materialien bestehen.

Bei der kieferorthopädischen Apparatur nach der Erfindung ist das als ein als vorgefertigtes Bauelement ausgebildete Verstellelement zur Einstellung der Apparatur aus einem ersten Fertigungsmaterial ausgebildet. Das Verstellelement kann z. B. eine Schraube sein. Das Verstellelement muss nicht am Kiefer anliegen, sondern kann sich an weitere kieferorthopädisch wirksame Elemente anschliessen.

Bei einer Ausführungsform der Erfindung umfasst die kieferorthopädische Apparatur wenigstens ein drahtartiges Element als ein kieferorthopädisch wirksames Elemente, das sich über mehrere Zähne erstreckt und an die Kontur der Zähne angeformt ist. Das angeformte drahtartige Element folgt der Kontur der Zähne derart, dass das Element zumindest grösstenteils an der Aussenfläche der Zähne anliegt. Ein Druck, der von dem drahtartigen Elemente auf die Zähe ausgeübt wird, kann dadurch grossflächig auf die Zähne übertragen werden, wodurch die kieferorthopädische Wirkung präzise ausgeübt werden kann.

Weiter ist bei einer Ausführungsform einer kieferorthopädischen Apparatur der Erfindung ein Halteelement derart ausgebildet, dass es einen Zahn zumindest teilweise umgibt und an diesen angeformt, bzw. angepasst ist. Demnach liegt eine Anlagefläche des Halteelements zumindest grösstenteils unmittelbar auf der Aussenfläche eines Zahns an. Das Halteelement schmiegt sich somit an den Zahn an und eine Haltekraft wird grossflächig auf den Zahn übertragen. Vorzugsweise ist das Haltelement ringförmig ausgebildet und umgibt den Zahn vollständig.

Eine erfindungsgemässe kieferorthopädische Apparatur wird vorzugsweise durch ein Verfahren zur Herstellung einer kieferorthopädischen Apparatur nach der vorliegenden Erfindung gefertigt. Das erfindungsgemässe Verfahren umfasst wenigstens nachfolgende Schritte. Es wird ein elektronischer Datensatz für ein drei-dimensionales virtuelles Modell eines Kiefers, an dem die Apparatur angebracht werden soll, erstellt. Anschliessend wird wenigstens ein kieferorthopädisch wirksames Element der Apparatur virtuell am virtuellen Kiefermodell positioniert. Dabei wird ein oder mehrere vordimensionierte oder vorgefertigte Bauelemente ausgebildet als Verstellelement virtuell relativ zu den kieferorthopädische wirksamen Elementen beispielsweise durch Computersimulation am virtuellen Kiefermodell angeordnet. Gleichzeitig kann eine Formgebung des kieferorthopädisch wirksamen Elements festgelegt werden. Vorzugsweise wird das Element der Kontur des Kieferbereichs, an dem es angeordnet sein soll, angepasst. Zumindest eine Anlagefläche, mit der das kieferorthopädisch wirksame Element und somit die Apparatur am Kiefer anliegt, folgt somit wenigstens grösstenteils der Kieferkontur. So werden vorteilhaft beim virtuellen Positionieren die kieferorthopädisch wirksamen Elemente am virtuellen Kiefermodell angeformt. Danach wird ein elektronischer Datensatz eines drei-dimensionalen Modells der Apparatur erstellt. Dieser Datensatz umfasst die Positionierung des wenigstens einen kieferorthopädisch wirksamen Elements und, falls für die Apparatur vorgesehen, weitere Elemente, wie etwa Halteelemente. Letztlich wird die Apparatur durch ein additives Fertigungsverfahren gemäss dem Datensatz des Apparaturmodells gefertigt.

Das erfindungsgemässe Verfahren ist insbesondere für die Herstellung einer kieferorthopädischen Apparatur vorteilhaft, wie sie oben beschrieben wurde. Das Verfahren kann aber auch vorteilhaft zur Weiterbildung einer bestehenden Apparatur verwendet werden, wobei weitere Elemente durch additives Fertigungsverfahren hinzugefügt und/oder bestehende Elemente oder teile von Elementen durch ein abtragendes Fertigungsverfahren entfernt werden. Das Verfahren ist zur Herstellung von kieferorthopädischen Apparaturen mit dem Zweck, Zähne zu bewegen, wie einer Bewegung der Zähne auf dem Kiefer alleine, oder einer Bewegung der Kiefer zu einander, oder unerwünschte Zahnbewegungen zu verhindern, geeignet. Es können z. B. herausnehmbare Zahnspangen, Retainer und der gleichen, aber auch Elemente für befestigte Apparaturen gefertigt werden.

Die Erstellung und Verarbeitung der elektronischen Datensätze wird von einem Computer ausgeführt, der u. a. einen geeigneten Algorithmus zur Umsetzung von Bilddaten des Kiefers in Modelldaten und zur Positionierung von Elementen relative zum virtuellen Kiefermodell aufweist. Die Bilddaten des Kiefers werden z. B. durch einen Scanner (intraoral oder extraoral), eine Kamera, eine ionisierende- oder andere nicht licht-basierende bildgebende Technologie (CT, DVT, MRI) erfasst, wie aus dem Stand der Technik bekannt. Der Computer übermittelt den Datensatz für die Apparatur an eine Steuereinheit einer Fertigungsanlage für additive oder abtragende Fertigungsverfahren.

Als additives Fertigungsverfahren wird z. B. selektives Laser-Melting, selektives Laser-Sintering oder Rapid-Prototyping verwendet. Bei additiven Fertigungsverfahren wird ein drei-dimensionales Objekt hergestellt, indem einzelne Materialschichten in einer Ebene entsprechend der Form des Objekts aufgetragen und gehärtet werden. So wird das Objekt schichtweise aufgebaut, wobei sich die einzelnen Schichten zu einem Ganzen verbinden und das Objekt in einem Stück geformt ist. Durch einen Wechsel des Fertigungsmaterials kann das Objekt auch Bereiche unterschiedlichen Materials aufweisen. Ein additives Fertigungsverfahren hat den Vorteil, dass weniger Material zur Herstellung der Apparatur erforderlich ist. Als Material, aus dem die Apparatur aufgebaut wird, wird biologisch verträgliches Material verwendet, das in geeigneter Form bereitgestellt werden kann, um die einzelnen Schichten der Apparatur auszubilden. Das Material kann z. B. in flüssiger Form oder in Pulverform vorliegen. Als Metall kann z. B. Kobaltchrom oder Legierungen daraus verwendet werden. Vorteilhaft können zur Fertigung der Apparatur unterschiedliche Fertigungsmaterialien für unterschiedliche Elemente verwendet und aneinander gefügt werden. Dadurch können z. B. drahtartige Elemente aus Metall und Kieferplatten aus Kunststoff gefertigt werden. Es können für verschiedene Bereiche der Apparatur Materialen verwendet werden, die nach dem Aushärten unterschiedliche Anforderungen an Elastizität, Härte und dergleichen erfüllen.

Als abtragendes Fertigungsverfahren wird z. B. ein Laser-Cutting-Verfahren verwendet, wobei entsprechend dem drei-dimensionalen Model von einem Rohling Material abgetragen wird, um die gewünschte Form zu erhalten.

Ein Vorteil dieses Herstellungsverfahrens ist es, dass dem Patienten die unangenehmen Abformungen mittels Alginat, Silikon oder ähnlichem erspart wird. Zudem bietet die grosse Variabilität der Formgebung mittels der virtuellen Positionierung der Elemente die Möglichkeit die Zahnspangen für den Patienten so angenehm wie möglich zu gestalten. Dicke und Festigkeit der Elemente und der gesamten Apparatur können optimal angepasst werden und es kann eine perfekte Passgenauigkeit für die Verbindung Zahn-Zahnspange erreicht werden.

Bei dem erfindungsgemässen Verfahren werden mehrere kieferorthopädisch wirksame Elemente der Apparatur unabhängig voneinander positioniert und in einem Apparaturmodell zusammengefasst. In dem Apparaturmodell sind somit die mehreren kieferorthopädisch wirksamen Elemente zu einem Stück zusammengefügt. Vorzugsweise werden alle kieferorthopädisch wirksamen Elemente und auch Halteelemente zum Halten der Apparatur am Kiefer und sonstige Elemente am virtuellen Kiefermodell positioniert, in den Apparaturdatensatz aufgenommen und durch das additive, bzw. abtragende Fertigungsverfahren gefertigt. Somit kann die Apparatur schnell und passgenau gefertig werden.

Bei dem erfindungsgemässen Verfahren werden eines oder mehrere vordimensionierte oder vorgefertigte Bauelemente, wie z. B. Verstellelemente in Form von Schrauben, virtuell relativ zu den kieferorthopädisch wirksamen Elementen positioniert und in den elektronischen Datensatz für das Apparaturmodell aufgenommen werden. Bei der additiven Fertigung der Apparatur wird dann das Fertigungsmaterial des wenigstens einen kieferorthopädisch wirksamen Elements und des wenigstens einen Halteelements schichtweise um das Bauteil verteilt und gehärtet, sodass dieses in der Apparatur fest aufgenommen ist. Dabei ist es auch möglich, dass durch die Strahlung, die zum Aushärten verwendet wird, sich das Fertigungsmaterial chemisch mit dem Material des Bauelements verbindet. Die Apparatur kann somit beliebig mit Bauteilen z. B. in Form von herkömmlichen zahntechnischen Geräten erweitert werden.

Bei noch einer weiteren Variante des erfindungsgemässen Verfahrens können nach Erstellung der Apparatur weitere Elemente durch ein additives Fertigungsverfahren hinzugefügt und/oder bestehende Elemente oder Teile von Elementen durch ein abtragendes Fertigungsverfahren entfernt werden. Dadurch kann die Apparatur an neue Anforderungen angepasst werden oder nach der Fertigstellung korrigiert werden, falls erforderlich. Hierfür wird die bestehende Apparatur in den Datensatz für die Modellapparatur aufgenommen und die weiteren Elemente virtuell ergänzt oder es werden Bereiche virtuell entfernt, die abgetragen werden sollen.

Bei dem Verfahren wird vorzugsweise beim Fertigen der Apparatur die Apparatur derart ausgerichtet oder angeordnet, dass bei der Schichtung, respektive dem Aushärten des Fertigungsmaterials keine zu grosse Hitze im Umfeld eines Schmelzortes entsteht, welche bereits vollendeten Anteilen der Apparatur schaden könnten.

Es ist ein Vorteil des Verfahrens nach der Erfindung, dass die Apparatur mit Elementen aus verschiedenen Metallen, wie etwa Kobaltchrom und Nickel-Titanium, hergestellt werden. Dies kann entweder durch Anfügen bei der Ausarbeitung, durch direktes Verbinden beim Entstehungsprozess oder durch lokales ändern der Materialzusammensetzung bei dem Herstellungsprozess geschehen. So können z. B. Elemente wie Finger-Federn an der Apparatur vorgesehen werden. Die Apparatur kann auch mittels erfasster 3D-Daten von bestehenden Zahnspangen, z. B. von Molarenbändern, direkt mit diesen verbunden werden, wobei Verbindungsteile entsprechend der Formgebung ausgestaltet und im Apparaturmodel aufgenommen werden. Weiter kann die Apparatur mit Kunststoffelementen erweitert werden, die entweder konventionell oder durch Rapid-Prototyping hergestellt wurden, wie etwa Lip-Bumper mit einem labialen Kunststoff-Schild. Die Apparatur kann auf skelettalen Verankerungen befestigt werden, wie z. B. auf einem Mini-Implantat System.

Die Erfindung wurde an Hand mehrerer Beispiele dargestellt. Die einzelnen technischen Merkmale eines Beispiels kann durchaus auch in Kombination mit einem anderen Beispiel mit den dargelegten Vorteilen verwendet werden. Die Beschreibung der erfindungsgemässen technischen Merkmale ist daher nicht auf das jeweilige Beispiel beschränkt.

Vorteilhafte Ausführungsformen der Erfindung werden im Folgenden anhand der Zeichnungen dargestellt, die lediglich zur Erläuterung dienen und nicht einschränkend auszulegen sind. Aus den Zeichnungen offenbar werdende Merkmale der Erfindung sollen einzeln und in jeder Kombination als zur Offenbarung der Erfindung gehörend betrachtet werden. In den Zeichnungen zeigen:
Fig. 1 einen Ausschnitt aus einer Modellierung einer ersten kieferorthopädischen Apparatur,
Fig. 2 die erste kieferorthopädische Apparatur aus der Modellierung gemäss Figur 1,
Fig. 3 eine zweite kieferorthopädische Apparatur in Form eines Retainers,
Fig. 4 einen Ausschnitt aus einer Modellierung einer zweiten kieferorthopädischen Apparatur mit einem Verstellelement und
Fig. 5 die zweite kieferorthopädische Apparatur aus der Modellierung gemäss Figur 4.

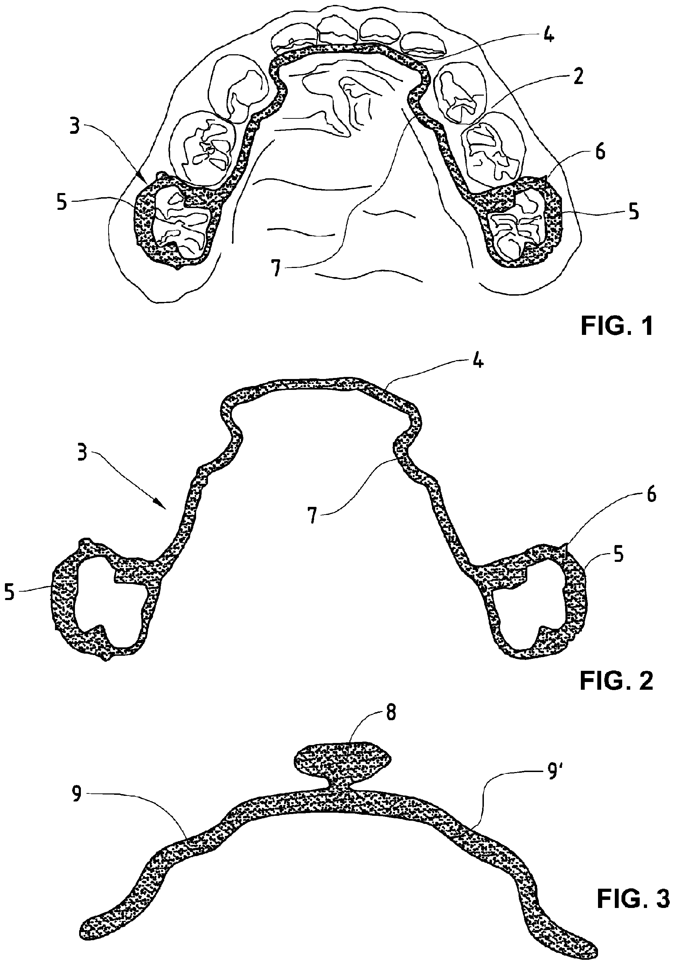

Figur 1 zeigt einen Ausschnitt aus einer Abbildung bei der Modellierung einer ersten kieferorthopädischen Apparatur 1, wie sie in Figur 2 gezeigt ist und nicht der erfindungsgemässen Apparatur entspricht. In Figur 1 ist ein Abbild eines drei-dimensionalen Kiefermodells 2 eines Unterkiefers mit Zähnen gezeigt, wie es durch einen elektronischen Datensatz für das Kiefermodell festgelegt ist. Der Datensatz wurde aus Abbildungen gewonnen, die im Mund eines Patienten aufgenommen wurden. Weiter ist ein Abbild eines drei-dimensionalen Modells der Apparatur, dem Apparaturmodell, 3 gezeigt, wie es durch einen elektronischen Datensatz für das Apparaturmodell festgelegt ist. Das Apparaturmodell 3 umfasst ein drahtartiges kieferorthopädisch wirksames Element 4, das sich auf der Innenseite des Kiefers entlang mehrerer Zähne erstreckt. Das kieferorthopädisch wirksame Element 4 liegt auf der Innenseite der Schneidezähne an diesen an und folgt der Kontur dieser Zähne, um sich an ihnen abzustützen. Weiter weist das drahtartige Element 4 eine Spannungsschlaufe 7 auf. Die seitlichen Bereiche des Elements 4 entlang der Backenzähne verlaufen in einem Abstand zu den Zähnen. An den beiden Enden des kieferorthopädisch wirksamen Elements 4 ist jeweils ein Halteelement 5 vorgesehen, das als ringförmiges Element einen Backenzahn umgibt. Die Haltelemente 5 liegen rings um den Zahn mit einer Auflagefläche am Zahn an, so dass die Auflagefläche an die Kontur des Zahns angepasst ist. Das Apparaturmodell zeigt, wie das kieferorthopädisch wierksame Element 4 und die Haltelemente 5 am Kiefermodell 2 positioniert sind, damit sich die Apparatur an den Schneidezähnen und den hinteren Backenzähnen abstützt und durch die Spannungsschlaufe 7 einen Druck ausüben kann. Zudem weisen die Halteelemente 5 an ihren Aussenfläche kleine Vorsprünge 6 auf, die als Angriffspunkte beim Lösen der Apparatur vom Kiefer dienen.

Grundsätzlich ist es möglich zusätzlich zu den Elementen, die für die Apparatur vorgesehen sind, Hilfselemente vorzusehen, welche die Herstellung mittels eines additiven Fertigungsverfahrens erleichtern. Die Hilfselemente werden in den Datensatz für das Apparaturmodell aufgenommen und bei der Fertigung mit produziert. Anschliessend werden die Hilfselemente entfernt, beispielsweise durch ein abtragendes Fertigungsverfahren. Als Hilfselemente können z. B. Stege oder Verstrebungen vorgesehen werden, welche die rechte und linke Kieferseite der Apparatur verbinden.

Figur 2 zeigt die kieferorthopädische Apparatur 1. Die Apparatur ist aus Metall. Es ist klar ersichtlich, dass die Apparatur 1 einstückig ist. Sowohl das drahtartige kieferorthopädisch wirksame Element 4 als auch die beiden Halteelemente 5 sind übergangslos als ein Stück geformt. Mit Hilfe des Verfahrens ist es möglich, verschiedene Bereich des drahtartigen Elements 4 mit unterschiedlichen Durchmessern oder Querschnitten auszubilden. Beispielsweise ist es vorteilhaft, wenn die seitlichen Bereiche und die Bereich an den Schneidezähnen einen ovalen Querschnitt aufweisen, wodurch das Element in diesen Bereichen steifer ist, und im Bereich der Spannungsschlaufe 7 eine runden Querschnitt vorzusehen, wodurch diese leichter biegsam ist.

Figur 3 zeigt einen nicht erfindungsgemässen Retainer, der dazu dient, eine unerwünschte Zahnbewegung zu verhindern. Der Retainer weist ein Halteelement 8 und zwei sich zu beiden Seiten des Halteelements 8 erstreckende kieferorthopädisch wirksame Elemente 9 und 9' auf. Die kieferorthopädisch wirksamen Elemente 9 und 9' sind an die Kontur der Zähne angepasst und halten diese in ihrer Position. Die Kontaktfläche zu den Zähnen ist jeweils abgeflacht für eine optimale Haftfläche, sodass der Durchmesser zwischen den Zähnen von rund zu halbrund bei den Zahnflächen variiert. Das Halteelement 8 dient der optimalen Positionierung im Munde des Patienten und wird nach dem Befestigen an den Zähnen abgetrennt. Der Retainer ist ebenfalls einstückig ausgebildet.

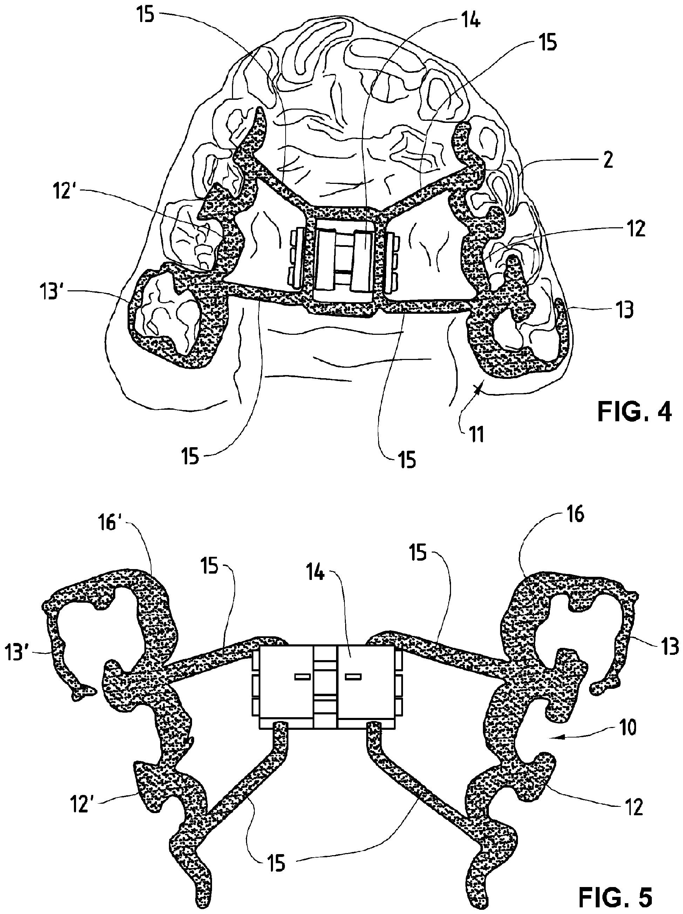

Figur 4 zeigt einen Ausschnitt aus einer Abbildung bei der Modellierung einer erfindungsgemässen kieferorthopädischen Apparatur 10, wie sie in Figur 5 gezeigt ist. In Figur 4 ist wiederum ein Abbild eines drei-dimensionalen Kiefermodells 2 gezeigt, in diesem Fall eines Oberkiefers. Die Abbildung zeigt ein Apparaturmodell 11, das auf der rechten und der linken Seite des Kiefers jeweils ein kieferorthopädisch wirksames Element 12 und 12' umfasst, die sich an die Innenseiten der Backenzähne anschmiegen und der Kontur der Zähne folgen. Um den hintersten Backenzahn sind die kieferorthopädisch wirksamen Element 12 und 12' mit Halteelementen 13 und 13' versehen, die beinahe vollständig um den Zahn verlaufen und auf diesem passgenau aufliegen. Weiter weist das Apparaturmodell 11 ein Verstellelement 14 in Form einer Dehnschraube auf. Das Verstellelement 14 ist durch drahtartige Verbindungselemente 15 mit den kieferorthopädisch wirksamen Elementen 12 und 12' verbunden. Das Verstellelement 14 liegt als vorgefertigtes Bauteil vor, wie es aus dem Stand der Technik bekannt ist. Von dem Bauteil wurde eine graphische Abbildung gemacht und die daraus gewonnen Bilddaten wurden in den Datensatz für das Apparaturmodell 11 integriert. Bei der Herstellung der kieferorthopädischen Apparatur 10 mit dem erfindungsgemässen Verfahren, wird das Bauteil für die Verstellschraube während des additiven Fertigungsprozesses in diesen Prozess mit einbezogen. Hierfür wird das Bauteil an der vorgesehenen Position gelagert und der schichtweise Aufbau der Apparatur erfolgt um das Bauteil, wobei Fertigungsmaterial auch mit dem Bauteil verbunden und mit diesem verhärtet wird.

Figur 5 zeigt die fertige kieferorthopädische Apparatur 10 gemäss der Erfindung. In Figur 5 sind eine Auflageflächen 16 und 16' der kieferorthopädisch wirksamen Elemente 12 und 12' und der Halteelemente 13 und 13' ersichtlich, mit welchen die Apparatur 10 auf den Zähnen eines Patienten aufliegt. Es ist klar zu erkennen, dass das Profil der Auflageflächen 16 und 16' der Kontur der Zähne entspricht, wie sie durch das Kiefermodell erfasst wurde.

Die Ausführungsform der kieferorthopädischen Apparatur gemäss den Figuren 4 und 5 zeigt eine Apparatur, die gemäss dem Verfahren zur Herstellung einer kieferorthopädischen Apparatur nach der vorliegenden Erfindung gefertigt ist. Anstelle das Verstellelement 14 als vorgefertigtes Bauteil in die Apparatur gemäss der Erfindung zu integrieren, kann das Verstellelement durch das additive Fertigungsverfahren gleichzeitig mit den kieferorthopädisch wirksamen Element 12 und 12' und den Verbindungselementen 15 gefertigt werden. Die auf diese Weise hergestellte kieferorthopädische Apparatur ist einstückig ausgebildet. Alle Elemente der Apparatur sind im selben Arbeitsschritt produziert. Dennoch ist es denkbar, z. B. die Verstellschraube aus einem anderen Fertigungsmaterial als die kieferorthopädisch wirksamen Elemente zu fertigen.

## Patentansprüche

1. Kieferorthopädische Apparatur (10) mit wenigstens einem kieferorthopädisch wirksamen Element (12, 12'), einem als vorgefertigtes Bauelement ausgebildetes Verstellelement (14) und wenigstens einem Halteelement (13, 13') zum Halten der Apparatur (10) am Kiefer, **dadurch gekennzeichnet, dass** die Apparatur (10) derart einstückig ausgebildet ist, dass die Apparatur (10) verschiedene Bereiche aufweist, welche den kieferorthopädisch wirksamen Elementen (12, 12') und den Halteelementen (13, 13') entsprechen und unmittelbar in einander übergehen, wobei das Verstellelement (14) zur Einstellung der Apparatur (10) aus einem ersten Fertigungsmaterial gefertigt ist, und wobei das wenigstens eine kieferorthopädisch wirksame Element (12, 12') und das wenigstens eine Halteelement (13, 13') aus einem zweiten Fertigungsmaterial gefertigt sind, welches schichtweise um das Verstellelement (14) verteilt und gehärtet ist.

2. Kieferorthopädische Apparatur (10) nach Anspruch 1, **dadurch gekennzeichnet, dass** die verschiedenen Bereiche der Apparatur (10) aus unterschiedlichen Fertigungsmaterialien bestehen, die ohne weitere Verbindungsmittel aneinander geformt sind.

3. Kieferorthopädische Apparatur (10) nach einem der vorhergehenden Ansprüche, **dadurch gekennzeichnet, dass** wenigstens eines der kieferorthopädisch wirksamen Elemente ein drahtartiges Element ist, das sich über mehrere Zähne erstreckt und an die Kontur der Zähne angeformt ist.

4. Kieferorthopädische Apparatur nach einem der vorhergehenden Ansprüche, **dadurch gekennzeichnet, dass** ein Halteelement (13) derart ausgebildet ist, dass es einen Zahn zumindest teilweise umgibt und an diesen angeformt ist.

5. Kieferorthopädische Apparatur nach einem der Ansprüche 1 bis 4, **dadurch gekennzeichnet, dass** das zweite Fertigungsmaterial chemisch mit dem als Verstellelement (14) ausgebildeten Bauelement verbunden ist.

6. Verfahren zur Herstellung einer kieferorthopädischen Apparatur (10) nach einem der Ansprüche 1 bis 5 umfassend folgende Schritte:
- Erstellen eines elektronischen Datensatzes für ein virtuelles drei-dimensionales Modell (2) eines Kiefers, an dem die Apparatur angebracht werden soll,
- virtuelles Positionieren eines oder mehrerer kieferorthopädisch wirksamer Elemente (12, 12') der Apparatur (10) und wenigstens eines Halteelements (13, 13') zum Halten der Apparatur (10) am Kiefer am virtuellen Kiefermodell (2),
- virtuelles Positionieren eines oder mehrerer vordimensionierter oder vorgefertigter Bauelemente ausgebildet als Verstellelement (14) relativ zu den kieferorthopädisch wirksamen Elementen (12, 12'),
- Erstellen eines elektronischen Datensatzes eines drei-dimensionalen Modells (11) der Apparatur (10), und
- Fertigen der Apparatur (10) durch ein additives Fertigungsverfahren gemäss dem Datensatz des Apparaturmodells.

7. Verfahren nach Anspruch 6, **dadurch gekennzeichnet, dass** die mehreren kieferorthopädisch wirksamen Elemente (12, 12') der Apparatur unabhängig voneinander positioniert und zu einem Apparaturmodell (11) zusammengefügt werden.

8. Verfahren nach einem der vorhergehenden Ansprüche 6 und 7, **dadurch gekennzeichnet, dass** nach Erstellung der Apparatur (10) weitere Elemente durch ein additives Fertigungsverfahren hinzugefügt und/oder bestehende Elemente oder Teile von Elementen durch ein abtragendes Fertigungsverfahren entfernt werden.

9. Verfahren nach einem der vorhergehenden Ansprüche 6 bis 8, **dadurch gekennzeichnet, dass** beim Fertigen der Apparatur (10) die Apparatur (10) derart ausgerichtet ist, dass bei einem Aushärten aufeinander folgender Materialschichten des additiven Fertigungsverfahrens entstehende Hitze von bereits gefertigten Anteilen der Apparatur abgeschirmt oder abgeleitet wird.

10. Verfahren nach einem der vorhergehenden Ansprüche 6 bis 9, **dadurch gekennzeichnet, dass** bei einem additiven Fertigungsverfahren im selben Fertigungsprozess zur Fertigung der Apparatur (10) unterschiedliche Materialien für unterschiedliche Elemente verwendet und aneinander gefügt werden.

11. Verfahren nach einem der vorhergehenden Ansprüche 6 bis 10, **dadurch gekennzeichnet, dass** beim virtuellen Positionieren kieferorthopädisch wirksamer Elemente (12, 12') diese Elemente am virtuellen Kiefermodell (2) angeformt werden.

## Claims

1. Orthodontic apparatus (10) with at least one orthodontically effective element (12, 12'), an adjustment element (14) designed as prefabricated component and at least one holding element (13, 13') for holding the apparatus (10) on the jaw, **characterized in that** the apparatus (10) is designed in one piece in such a way that the apparatus (10) has different regions which correspond to the orthodontically effective elements (12, 12') and the holding elements (13, 13') and merge directly into one another, whereby the adjustment element (14) for adjusting the apparatus (10) is made of a first production material, and whereby the at least one orthodontically effective element (12, 12') and the at least one holding element (13, 13') are made of a second production material, which is distributed in layers around the adjustment element (14) and is hardened.

2. Orthodontic apparatus (10) according to claim 1, **characterized in that** the different regions of the apparatus (10) are made of different production materials that are shaped with respect to one another without further connecting means.

3. Orthodontic apparatus (10) according to one of the preceding claims, **characterized in that** at least one of the orthodontically effective elements is a wire-type element, which extends over a plurality of teeth and is shaped to the contour of the teeth.

4. Orthodontic apparatus according to one of the preceding claims, **characterized in that** a holding element (13) is designed in such a way that it surrounds a tooth at least partially and is shaped to this tooth.

5. Orthodontic apparatus according to one of the claims 1 to 4, **characterized in that** the second production material is chemically bonded with the component designed as adjustment element (14).

6. Method of producing an orthodontic apparatus (10) according to one of the claims 1 to 5 comprising the following steps:
- producing an electronic data set for a virtual three-dimensional model (2) of a jaw, to which the apparatus is supposed to be attached,
- virtual positioning, on the virtual jaw model (2), of one or more orthodontically effective elements (12, 12') of the apparatus (10) and at least one holding element (13, 13') for holding the apparatus (10) on the jaw,
- virtual positioning of one or more predimensioned or prefabricated components designed as adjustment element (14) relative to the orthodontically effective elements (12, 12')
- producing an electronic data set for a three-dimensional model (11) of the apparatus (10), and
- producing the apparatus (10) by an additive manufacturing method according to the data set of the apparatus model.

7. Method according to claim 6, **characterized in that** the plurality of orthodontically effective elements (12, 12') of the apparatus are positioned independently of one another and are joined together into an apparatus model (11).

8. Method according to one of the preceding claims 6 and 7, **characterized in that** after production of the apparatus (10) further elements are added by an additive manufacturing method and/or existing elements or parts of elements are removed by a removal production method.

9. Method according to one of the preceding claims 6 to 8, **characterized in that** when producing the apparatus (10), the apparatus (10) is aligned in such a way that with a hardening of consecutive material layers of the additive manufacturing method heat arising from already produced portions of the apparatus is shielded or discharged.

10. Method according to one of the preceding claims 6 to 9, **characterized in that** with an additive manufacturing method in the same manufacturing method for producing the apparatus (10) different materials are used for different elements and are joined together.

11. Method according to one of the preceding claims 6 to 10, **characterized in that** with the virtual positioning of orthodontically effective elements (12, 12') these elements are shaped on the virtual jaw model (2).

## Revendications

1. Appareil orthodontique (10) comprenant au moins un élément orthodontiquement efficace (12, 12'), un élément de réglage (14) conçu sous la forme d'un composant préfabriqué et au moins un élément de maintien (13, 13') pour maintenir l'appareil (10) sur la mâchoire, **caractérisé en ce que** l'appareil (10) est réalisé de façon monobloc de telle sorte que l'appareil (10) présente différentes zones qui correspondent aux éléments orthodontiquement efficaces (12, 12') et aux éléments de maintien (13, 13') et se fondent directement l'un dans l'autre, l'élément de réglage (14) pour l'ajustement de l'appareil (10) étant réalisé en un premier matériau de production, et au moins l'élément orthodontiquement efficace (12, 12') et au moins l'élément de maintien (13, 13') étant réalisés en un deuxième matériau de production, qui est disposé en couches autour de l'élément de réglage (14) et durci.

2. Appareil orthodontique (10) selon la revendication 1, **caractérisé en ce que** les différentes zones de l'appareil (10) sont constituées de différents matériaux de production qui sont formés de manière à se jouxter l'un l'autre sans autre moyen de liaison.

3. Appareil orthodontique (10) selon l'une des revendications précédentes, **caractérisé en ce qu'**au moins l'un des éléments orthodontiquement efficaces est un élément de type fil de fer, qui s'étend sur une pluralité de dents et possède la forme du contour des dents.

4. Appareil orthodontique (10) selon l'une des revendications précédentes, **caractérisé en ce qu'**un élément de maintien (13) est conçu de telle sorte qu'il entoure une dent au moins partiellement et possède la forme de cette dent.

5. Appareil orthodontique (10) selon l'une des revendications précédentes 1 à 4, **caractérisé en ce que** le deuxième matériau de production est relié chimiquement avec le composant conçu comme élément de réglage (14).

6. Procédé de fabrication d'un appareil orthodontique (10) selon l'une des revendications précédentes 1 à 5 comprenant les étapes suivantes :
- la production d'un ensemble de données électroniques pour un modèle virtuel tridimensionnel (2) d'une mâchoire, auquel l'appareil est censé être fixé,
- le positionnement virtuel, sur le modèle de mâchoire virtuel (2), d'un ou plusieurs éléments orthodontiquement efficaces (12, 12') de l'appareil (10) et d'au moins un élément de maintien (13, 13') pour le maintien de l'appareil (10) sur la mâchoire,
- le positionnement virtuel d'un ou plusieurs composants prédimensionnés ou préfabriqués réalisés comme élément de réglage (14) vis-à-vis des éléments orthodontiquement efficaces (12, 12'),
- la production d'un ensemble de données électroniques pour un modèle tridimensionnel (11) de l'appareil (10), et
- la production de l'appareil (10) par un procédé de fabrication additive selon l'ensemble de données du modèle d'appareil.

7. Procédé selon la revendication 6, **caractérisé en ce que** la pluralité d'éléments orthodontiquement efficaces (12, 12') de l'appareil sont positionnés indépendamment les uns des autres et sont ensuite assemblés dans un modèle d'appareil (11).

8. Procédé selon l'une des revendications précédentes 6 ou 7, **caractérisé en ce qu'**après la production de l'appareil (10) d'autres éléments sont ajoutés par un procédé de fabrication additive et/ou des éléments existants ou des parties d'éléments sont supprimées par un procédé de fabrication par enlèvement.

9. Procédé selon l'une des revendications précédentes 6 à 8, **caractérisé en ce que**, lors de la production de l'appareil (10), l'appareil (10) est orienté de telle sorte qu'avec un durcissement de couches de matériaux consécutives du procédé de fabrication additive, la chaleur provenant des parties déjà produites de l'appareil est protégée ou évacuée.

10. Procédé selon l'une des revendications 6 à 9, **caractérisé en ce que** lors d'un procédé de fabrication additive du même procédé de fabrication pour la réalisation de l'appareil (10), différents matériaux sont utilisés pour différents éléments et sont joints les uns aux autres.

11. Procédé selon l'une des revendications précédentes 6 à 10, **caractérisé en ce que** lors du positionnement virtuel des éléments orthodontiquement efficaces (12, 12'), ces éléments sont formés sur le modèle de mâchoire virtuel (2).
